# FASCICULE DE BREVET EUROPEEN

(11) **EP 0 805 136 B1**
(45) Date de publication et mention de la délivrance du brevet: **25.07.2001**
(21) Numéro de dépôt: 97400754.4
(22) Date de dépôt: 02.04.1997
(51) Int. Cl.: C07C 17/20, C07C 19/08

(54) **Procédé de fabrication du difluorométhane**
Verfahren zur Herstellung von Difluormethan
Process for the preparation of difluoromethane

(30) Priorité: 29.04.1996 FR 9605369
(43) Date de publication de la demande: 05.11.1997
(73) Titulaire: Atofina, 92800 Puteaux (FR)
(72) Inventeur: Garrait, Dominique, 69390 Millery (FR); Guiraud, Emmanuel, 69230 Saint Genis Laval (FR)
(74) Mandataire: Leboulenger, Jean

(56) Documents cités:
- EP-A- 0 751 108
- WO-A-94/21579
- WO-A-95/12563
- PATENT ABSTRACTS OF JAPAN vol. 018, no. 668 (C-1289), 16 Décembre 1994 & JP 06 263657 A (SHOWA DENKO KK), 20 Septembre 1994,
- DATABASE WPI Section Ch, Week 7514 Derwent Publications Ltd., London, GB; Class E16, AN 75-23265W XP002022129 & JP 49 134 612 A (ASAHI GLASS CO LTD) , 25 Décembre 1974

## Description

La présente invention concerne le domaine des hydrocarbures fluorés et a plus particulièrement pour objet un procédé continu de fabrication du difluorométhane à partir de chlorure de méthylène et d'acide fluorhydrique HF.

Depuis que les chlorofluorocarbures (CFC) ont été identifiés comme l'un des responsables de l'accélération de la dégradation de la couche d'ozone stratosphérique, les acteurs politiques et industriels se sont engagés de manière irréversible dans un processus de substitution des CFC. Ce processus de substitution concerne des secteurs industriels essentiels tels que la chaîne du froid alimentaire, l'isolation des bâtiments, la climatisation, la micro-électronique, etc.

Les substituts envisagés sont des composés fluorés contenant des atomes d'hydrogène, mais pas d'atomes de chlore. L'un de ces composés, sans effet sur la couche d'ozone, est le difluorométhane qui est connu dans le métier sous la désignation F32 et est principalement destiné à remplacer le F22 (chlorodifluorométhane) et le R502 (mélange azéotropique de F22 et de chloropentafluoroéthane) dans le domaine de la réfrigération, de l'air conditionné et autres applications. L'intérêt est donc important de développer un procédé le plus simple possible pour produire le F32 en quantités importantes et économiquement compétitives.

L'accès au F32 par fluoration en phase gazeuse du chlorure de méthylène, connu dans le métier sous la désignation F30, a déjà fait l'objet de brevets revendiquant l'utilisation de catalyseurs tels que Cr₂O₃, CrF₃, Cr/charbon, Ni/AlF₃...

Cependant, comme celle de la plupart des substituts aux CFC, la production de F32 pose de sérieux problèmes car elle génère beaucoup de sous-produits et impuretés qui, après séparation de l'HCl et du F32, se retrouvent soit dans l'HCl et le F32 récupérés, soit dans le flux à recycler comprenant majoritairement du F30 et du F31 (chlorofluorométhane).

Dans le cas de la production du F32 par fluoration du chlorure de méthylène, le problème le plus sérieux est posé par la génération, comme composé intermédiaire, de quantités importantes de F31 hautement toxique. Ces teneurs peuvent être de l'ordre de 20 % et il est donc impératif de limiter au maximum la circulation et le temps de séjour de ce composé dans l'installation ainsi que les opérations unitaires mettant en jeu des flux contenant du F31.

Dans la fluoration catalytique du F30, le taux de conversion du F30 en F32 est limité par la thermodynamique. Typiquement, pour un ratio molaire HF/Organiques égal à 3 à l'entrée du réacteur et une température réactionnelle de 300°C, l'équilibre thermodynamique correspond à des taux de conversion du F30 de 65 % et de l'HF de 43 %. Le flux sortant du réacteur contient donc majoritairement des réactifs non transformés (F30, F31 et HF) qu'il est primordial de recycler. Pour cela, on peut conformément aux techniques classiques séparer puis purifier les principaux constituants du flux sortant du réacteur, notamment les F30, F31 et l'HF non convertis pour en éliminer avant recyclage en réaction les impuretés gênantes (telles que des sous-produits organiques ou de l'eau) qui sont générées en réaction ou apportées par les matières premières et qui sont susceptibles d'entraîner une désactivation du catalyseur ou de générer des corrosions.

Lors de ce type de manipulation, les flux à traiter sont très concentrés en F31, ce qui nécessite un renforcement des mesures et matériels de sécurité et donc une augmentation des coûts.

Le recyclage direct du flux de l'HF, du F30 et du F31 au réacteur après séparation de l'HCl et du F32 produits, sans purification préalable, présente l'avantage de limiter les manipulations de flux concentrés en F31. C'est pourquoi, la plupart des brevets décrivant un procédé d'accès au F32 par fluoration du F30 avec de l'HF anhydre en phase gazeuse mentionnent le recyclage direct des produits non réagis (F30, F31, HF) au réacteur après séparation de l'HCl et du F32 produits (demandes de brevet JP 5-50953/93, WO 94/21579 et WO 95/12563).

Il est par ailleurs connu (demandes de brevet JP 51-82206 et JP 49-134612) que l'injection d'oxygène ou d'air ou de chlore en continu peut augmenter la durée de vie des catalyseurs de fluoration qui ont tendance à se cocker ou à cristalliser très rapidement. Cependant, lors de la synthèse du F32 en présence d'oxygène, classiquement utilisé pour le maintien de l'activité catalytique, le recyclage des produits bruts au réacteur dégrade considérablement et rapidement (moins de 100 heures) les performances du catalyseur par rapport à une marche sans recyclage.

C'est probablement pour cette raison que l'utilisation d'oxygène ou de chlore pour le maintien de l'activité du catalyseur lors de la fluoration en phase gazeuse du F30 n'est mentionnée dans aucun des brevets décrivant un procédé d'accès au F32 par fluoration du F30 avec l'HF anhydre en phase gazeuse avec recyclage direct des produits non réagis (F30, F31, HF) au réacteur après séparation de l'HCl et du F32 produits.

Il a maintenant été trouvé que l'injection de chlore avec les réactifs (F30 et HF) est non seulement plus efficace que l'injection d'oxygène pour stabiliser l'activité catalytique, mais autorise sans inconvénient le recyclage direct (sans purification) du flux des produits non réagis (F30, F31 et HF).

L'invention a donc pour objet un procédé continu de fabrication du F32 à partir de F30 et d'HF en phase gazeuse en présence d'un catalyseur de fluoration, caractérisé en ce que la réaction est effectuée en présence de chlore, et que le flux gazeux sortant du réacteur est soumis à une distillation pour séparer en tête un flux contenant la quasi-totalité de l'acide chlorhydrique et au moins 90 % du F32 produits par la réaction et en pied un flux contenant au moins 90 % des réactifs non transformés (F30, F31 et HF) présents dans le flux gazeux sortant du réacteur, et que l'on recycle directement au réacteur le flux récupéré en pied de distillation, sans aucune opération de purification.

Comme on pouvait s'y attendre, le recyclage des réactifs non convertis directement au réacteur, en l'absence de purification particulière du recyclat, conduit à un certain taux d'accumulation d'eau et de sous-produits organiques dans ce recyclat. En régime établi, la teneur en ces sous-produits organiques se stabilise dans un état stationnaire. Curieusement, la nature de ces sous-produits et leur teneur n'obèrent pas les performances du catalyseur :
- taux de conversion du F30 proche de l'état d'équilibre thermodynamique de la réaction 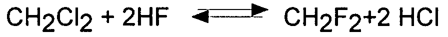
- sélectivité en F32 élevée, typiquement de l'ordre de 80 % molaire.

Dans la mise en oeuvre du procédé selon l'invention, ces performances (activité, sélectivité) restent stables pendant au moins 1000 heures ; ceci permet d'éviter des opérations fréquentes de remplacement ou de régénération du catalyseur, génératrices de coûts élevés en investissement et frais opératoires. En outre, le procédé selon l'invention est d'autant plus en sécurité qu'il n'implique pas d'opérations de purification du flux à recycler, et donc pas de production d'effluents contenant du F31 toxique.

Le catalyseur de fluoration à utiliser pour la mise en oeuvre du procédé selon l'invention peut être un catalyseur massique ou un catalyseur supporté, le support stable dans le milieu réactionnel étant, par exemple, un charbon actif, une alumine, une alumine partiellement fluorée, le trifluorure d'aluminium ou le phosphate d'aluminium. Par alumine partiellement fluorée, on entend une composition riche en fluor et contenant principalement de l'aluminium, du fluor et de l'oxygène dans des proportions telles que la quantité de fluor exprimée en AlF₃ constitue au moins 50 % du poids total. On utilise de préférence un catalyseur à base de chrome.

Parmi les catalyseurs massiques, on peut citer plus particulièrement l'oxyde de chrome III préparé selon l'une quelconque des méthodes connues de l'homme du métier (procédé sol-gel, précipitation de l'hydroxyde à partir des sels de chrome, réduction de l'anhydride chromique, etc...) et le trifluorure de chrome. Les dérivés de métaux tels que nickel, fer, vanadium (au degré d'oxydation III), manganèse, cobalt, zinc, peuvent également convenir seuls ou en association avec le chrome, sous forme de catalyseurs massiques, mais aussi sous forme de catalyseurs supportés. Il est également possible d'incorporer à ces catalyseurs ou à leur support des alcalinoterreux, des terres rares, du graphite ou de l'alumine pour en accroître la stabilité thermique ou mécanique. Lors de la préparation de catalyseurs associant plusieurs dérivés métalliques, les catalyseurs peuvent être obtenus par mélange mécanique ou par toute autre technique telle qu'une coprécipitation ou une co-imprégnation.

Les catalyseurs supportés ou massiques peuvent être employés sous forme de billes, d'extrudés, de pastilles ou même, si l'on opère en lit fixe, sous forme de morceaux. Lorsque l'on opère en lit fluide, on préfère utiliser un catalyseur sous forme de billes ou d'extrudés.

Comme exemples non limitatifs de catalyseurs, on peut mentionner :
- les microbilles d'oxyde de chrome obtenues par le procédé sol-gel comme décrit dans le brevet FR 2 501 062,
- les catalyseurs d'oxyde de chrome déposé sur charbon actif (brevet US 4 474 895), sur phosphate d'aluminium (brevet EP 55 958) ou sur fluorure d'aluminium (brevets US 4 579 974 et 4 579 976),
- les catalyseurs mixtes d'oxyde de chrome et de chlorure de nickel déposés sur fluorure d'aluminium (demande de brevet EP 0 486 333),
- les catalyseurs massiques à base d'oxyde de chrome cristallisé (demande de brevet EP 657 408),
- les catalyseurs massiques à base d'oxyde de chrome et de nickel (demande de brevet EP 0 546 883),
- les catalyseurs massiques à base d'oxyde de chrome et de vanadium (demande de brevet EP 0 657 409).

Les brevets précités dont le contenu est incorporé ici par référence décrivent largement le mode de préparation de ces catalyseurs, mais aussi leur mode d'activation, c'est-à-dire de transformation préalable du catalyseur en espèces actives stables par fluoration au moyen d'HF gazeux seul ou, plus généralement, mélangé à un gaz inerte tel que l'azote. Ce traitement est en général réalisé pendant une durée de 1 à 24 heures et à une température comprise entre 200 et 450°C. Durant cette activation, les oxydes métalliques servant de matière active (par exemple l'oxyde de chrome) ou de support (par exemple l'alumine) peuvent être partiellement ou complètement transformés en fluorures correspondants.

Sont plus particulièrement préférés les catalyseurs mixtes à base de chrome et de nickel décrits dans les demandes de brevet EP 0 486 333 et EP 0 546 883.

La réaction proprement dite du F30 avec l'HF en présence d'un catalyseur de fluoration peut être conduite dans un domaine de température compris entre 220 et 400°C, préférablement entre 240 et 350°C, avec un temps de contact compris entre 0,1 seconde et 60 secondes, préférablement de 1 à 20 secondes.

La pression sous laquelle la réaction peut être conduite est comprise entre la pression atmosphérique et 30 bars absolus. On opère préférablement sous une pression allant de 10 à 15 bars absolus, ce qui permet de réaliser économiquement la séparation de l'HCl anhydre du F32.

La quantité d'acide fluorhydrique utilisé est au moins égale à la stoechiométrie, mais le rapport molaire HF/Organiques à l'alimentation de la réaction est avantageusement compris entre 1 et 10, de préférence 2 à 5. On entend par Organiques, au sens du présent texte, les composés hydrocarbonés introduits dans le réacteur de fluoration. Ces composés comprennent le F30, le F31, éventuellement une faible proportion de F32 non séparé par distillation et diverses impuretés également de nature organique telles que le F20, le F21, le F22, le F112 et le F113.

Rapportée aux organiques alimentant la réaction, la quantité de chlore utilisée pour améliorer la durée de vie du catalyseur peut varier entre 0,1 et 5 % molaires. Le chlore peut être introduit dans la zone réactionnelle seul ou en mélange avec un inerte tel que l'azote.

L'emploi de chlore ne perturbe en rien la séparation en aval de la réaction et permet de recycler le brut réactionnel sans baisse d'activité du catalyseur. On atteint un régime stationnaire permettant de fonctionner en boucle durant au moins plusieurs centaines d'heures.

La réaction du F30 avec l'HF peut être réalisée dans différents types de réacteurs suivant le catalyseur utilisé, ses propriétés mécaniques et sa résistance à l'attrition. On peut opérer en lit fixe ou en lit fluide et dans un ou plusieurs réacteurs. Les matériaux utilisés doivent résister à la corrosion du milieu et doivent être, par exemple, de l'Inconel ou de l'Hastelloy.

Le flux gazeux sortant du réacteur de fluoration comprend principalement de l'HF, du F30, du F31, du F32 et de l'HCl. Conformément à la présente invention, ce flux gazeux est soumis à une séparation par distillation de façon à récupérer, d'une part, la quasi-totalité de l'HCl et au moins 90 % du F32 présents dans ce flux, et d'autre part, au moins 90 % du F30, du F31 et de l'HF qui sont recyclés directement en réaction.

Cette séparation peut être effectuée par distillation en une ou deux étapes, c'est-à-dire en séparant l'HCl puis le F32 ou directement, et plus simplement, en une étape en séparant l'HCl et le F32 en mélange. Dans ce cas on obtient en tête de distillation l'essentiel de l'HCl et du F32 et en pied l'essentiel du F30, F31 et de l'HF.

Cette distillation est préférablement réalisée dans une colonne en acier inoxydable qui peut être équipée de plateaux ou d'un garnissage. La distillation peut être menée sous une pression pouvant aller de 1 à 30 bars absolus, suivant la pression sous laquelle est effectuée la réaction de fluoration catalytique. La température d'alimentation du mélange réactionnel peut aller de 20 à 150°C. La température de tête dépend bien sûr de l'efficacité de séparation recherchée et varie en fonction de la pression ; elle est d'environ 0°C sous 12 bars absolus pour une pureté en F32 supérieure à 99,5 % mol.

A pression fixée, la température de tête sert à régler la teneur en F31 et HF du flux de tête tandis que le taux de rebouillage en pied sert à régler l'élimination de l'HCl et du F32. La proportion d'HF, passant en tête dépend essentiellement de la composition, à la pression considérée, des azéotropes formés avec le F31, le F32 et les composés de la série F20 comme le F22 (chlorodifluorométhane) et le F23 (trifluorométhane).

On observe que si l'on n'élimine pas l'essentiel de l'HCl et 90 % au moins du F32 produits en réaction, la productivité décroît en réaction. De même, le fait de ne pas séparer et recycler directement au moins 90 % du F30, F31 et de l'HF, présents en sortie de réaction, contraint inutilement et dangereusement à retraiter ces produits en aval de la boucle réactionnelle.

On opère de préférence la réaction et cette distillation sous une pression comprise entre environ 10 et 15 bars absolus. En effet, dans ces conditions, le mélange HCl/F32 est lui aussi séparable économiquement par distillation avec production d'acide chlorhydrique anhydre. Par contre, à une pression de l'ordre de 2 à 3 bars absolus, le mélange HCl/F32 issu de cette distillation doit généralement être traité à l'eau pour éliminer l'HCl.

Le flux des réactifs non convertis, obtenu en pied de la colonne de distillation, n'est soumis à aucun traitement particulier de purification ou d'élimination d'impuretés organiques ou minérales. Ce flux, majoritairement composé de F30, de F31 et d'HF, contient donc diverses impuretés organiques, des traces d'eau (de l'ordre de 1000 ppm en poids ou moins) et éventuellement une faible proportion des produits de réaction (F32 et HCI) non séparés. Ce flux est directement recyclé après décantation au réacteur de fluoration. Les réactifs frais (F30 et HF) sont par ailleurs alimentés en un point quelconque de l'ensemble réaction-séparation-recyclage, dans des proportions permettant de compenser la production nette de F32 et d'HCl.

Les réactifs frais peuvent être introduits soit avant distillation en aval de la réaction pour refroidir les gaz, soit dans le flux des réactifs non convertis recyclés en réaction.

Les F31, F30 et l'HF éventuellement présents en faible proportion dans le flux de F32 et d'HCI issu en tête de distillation peuvent être séparés ultérieurement des produits de réaction par les méthodes connues en soi et recyclés au réacteur.

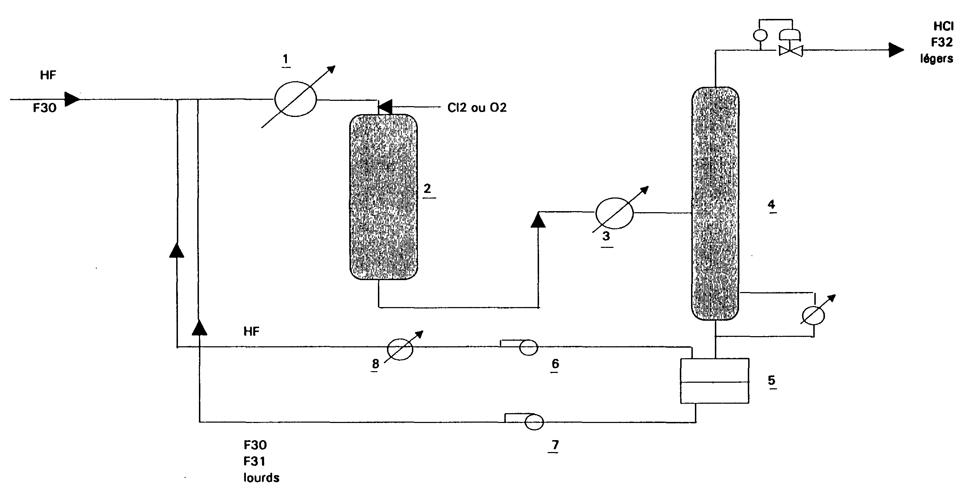

L'exemple suivant illustre l'invention sans la limiter. Cet exemple et les exemples comparatifs ci-après ont été réalisés sur une installation représentée sur la figure unique annexée. Cette installation comprend un réacteur (2) en Inconel d'un volume utile de 100 litres et une colonne de distillation (4) en inox 316 L ayant un diamètre intérieur de 150 mm, une hauteur de 6300 mm et munie d'un garnissage Multiknit en Inox 316 L.

Les réactifs frais et le flux des réactifs non convertis (recyclage) sont alimentés au réacteur après préchauffage dans un préchauffeur électrique (1).

En tête de la colonne de distillation, on récupère l'HCl, le F32 et les produits légers, et en pied les F30, F31, l'HF et les produits plus lourds. Ce flux de réactifs non convertis est décanté (5) puis recyclé au réacteur par l'intermédiaire de deux pompes (6) et (7) et d'un évaporateur (8) pour la phase HF.

A l'alimentation du préchauffeur (1), on effectue l'appoint en HF et F30 frais. L'alimentation en oxygène ou en chlore se fait à l'entrée du réacteur, selon qu'il s'agit d'un exemple comparatif ou d'un exemple illustrant l'invention.

L'HF utilisé, de qualité technique, a une pureté de 99,9 % poids contenant comme impureté principale de l'eau à hauteur de 1000 ppm maximum.

Le F30 utilisé a une pureté supérieure à 99,95 %poids.

### EXEMPLE 1 - Comparatif

Le catalyseur utilisé est un catalyseur à base de nickel et de chrome supportés sur alumine fluorée (teneur pondérale en AIF₃ supérieure à 78 %) préparé par imprégnation avec de l'acide chromique et du chlorure de nickel hexahydraté, puis réduction au méthanol. Ses principales caractéristiques physico-chimiques sont les suivantes :

| *-* ***composition chimique*** (pondérale) | |
|---|---|
| fluor | 58,6 % |
| aluminium | 25,9 % |
| nickel | 6,4 % |
| chrome | 6,0 % |

| ***- propriétés physiques*** | |
|---|---|
| surface BET | 52 m²/g |
| granulométrie | billes de 1 à 2 mm de diamètre. |

Ce catalyseur a été préalablement séché puis traité à 300°C au moyen d'un mélange d'acide fluorhydrique et d'azote.

La fluoration du F30 avec ce catalyseur a été effectuée, sans recyclage, dans les conditions opératoires suivantes :

| | |
|---|---|
| - température de réaction | 250°C |
| - pression | 12 bars absolus |
| - pas de recyclage | |
| - temps de contact | 5 secondes |
| - rapport molaire HF/F30 | 3 |
| - rapport molaire Cl₂/F30 à | 0,018 |
| l'alimentation du réacteur. | |

Les performances observées après 1050 heures dans ces conditions sont les suivantes :
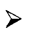 Conversion du F30 par passe : 62 %. (On entend par conversion le rapport entre le F30 consommé et le F30 entrant au réacteur).
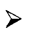 Sélectivité en F32 par passe : 75 % mol. (On entend par sélectivité en F32 le rapport molaire entre le F32 produit et le F30 consommé).
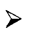 Productivité en F32 = 1450 g/h/litre de catalyseur.

### EXEMPLE 2

On opère comme à l'exemple 1 avec une charge neuve du même catalyseur Ni-Cr/AlF₃, mais avec recyclage direct du mélange brut contenant l'essentiel du F30, du F31 et de l'HF non transformés, après séparation des produits HCI et F32.

La fluoration du F30 avec ce catalyseur a été effectuée dans les conditions opératoires suivantes :

| ***a) Réaction*** | |
|---|---|
| - température de réaction | 250°C |
| - pression | 12 bars absolus |
| - temps de contact | 4 secondes |
| - rapport molaire HF/Organiques à l'alimentation du réacteur | 3 |
| - rapport molaire Cl₂/Organiques à l'alimentation du réacteur | 0,02 |

La colonne à distiller destinée à traiter le flux en sortie du réacteur a été réglée de la façon suivante

| ***b) Séparation*** | |
|---|---|
| - température d'alimentation (T_{A}) | 98°C |
| - température en tête (T_{T}) | 9°C |
| - chaleur fournie au bouilleur (Q_{B}) | 17 kW |
| - pression | 12 bars absolus. |

Ces conditions de fonctionnement ont permis d'obtenir en tête un flux de F32 et d'HCI et en pied un flux de F30, F31 et HF tels que :
- les teneurs molaires en F32 et HCI du flux de F30, F31 et HF recyclés étaient inférieures à 1 % pour le F32 et 100 ppm pour HCI,
- les teneurs molaires en F30, F31 et HF dans le flux de F32 et HCI étaient inférieures à 2000 ppm pour le F30, 2 % pour le F31 et 2 % pour l'HF.

Après 1000 heures de fonctionnement, les performances observées sont les suivantes :
***a)*** Conversion du F30 par passe : 61 %.
***b)*** Sélectivité en F32 par passe : 96 %.

Les impuretés principales dans le flux des produits de réaction récupéré en tête de colonne de distillation sont le F23 (2 % mol) et le F22 (1 % mol).
***c)*** Productivité en F32 = 1520 g/h/litre de catalyseur.
***d)*** Teneur en eau dans le flux recyclé : plusieurs analyses du flux recyclé ont été effectuées tout au long de cet essai afin de déterminer sa teneur en eau (méthode Karl Fischer). Les résultats ont montré qu'il n'y avait pas d'accumulation d'eau et que la teneur en H₂O dans le flux recyclé était inférieure à 400 ppm en poids.
***e)*** Teneur en impuretés organiques dans le recyclât : plusieurs analyses du flux recyclé ont été effectuées tout au long de cet essai afin de déterminer la teneur en impuretés organiques dans ce flux. Les résultats ont montré qu'il n'y avait pas d'accumulation et que la teneur en impuretés était stable. Cette teneur, exprimée en ppm poids par rapport au flux total recyclé, est inférieure à 20000 ppm. Les impuretés principales sont le F20 (0,2 % pds), le F21 (0,1 % pds), le F22 (0,3 % pds), le F112 (0,5 % pds) et le F113 (0,2 % pds).

### EXEMPLE 3 - Comparatif

On opère comme à l'exemple 1 avec une charge neuve du même catalyseur Ni-Cr/AlF₃, sans recyclage.

La fluoration du F30 avec ce catalyseur a été effectuée dans les conditions opératoires suivantes :

| | |
|---|---|
| - température de réaction | 300°C |
| - pression | 12 bar absolu |
| - temps de contact | 5 secondes |
| - rapport molaire HF/F30 | 3 |
| - rapport molaire O₂/F30 à | |
| l'alimentation du réacteur | 0,02 |

Après 400 heures de fonctionnement, les performances observées sont les suivantes :
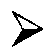 Conversion du F30 par passe : 40 %.
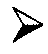 Sélectivité en F32 par passe : 59 %.
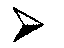 Productivité en F32 = 694 g/h/litre de catalyseur.

### EXEMPLE 4 - Comparatif

On opère comme à l'exemple 1 avec une charge neuve du même catalyseur Ni-Cr/AlF₃, avec recyclage direct du mélange brut contenant l'essentiel du F30, du F31 et de l'HF non transformés, après séparation des produits HCl et F32.

La fluoration du F30 avec ce catalyseur a été effectuée dans les conditions opératoires suivantes :

| ***a) Réaction*** | |
|---|---|
| - température de réaction | 300°C |
| - pression | 12 bars absolus |
| - temps de contact | 8 secondes |
| - rapport molaire HF/Organiques | |
| à l'alimentation du réacteur | 3 |
| - rapport molaire O₂/Organiques | |
| à l'alimentation du réacteur | 0,03 |

La colonne à distiller destinée à traiter le flux en sortie du réacteur a été réglée de la façon suivante :

| ***b) Séparation*** | |
|---|---|
| - température d'alimentation (T_{A}) | 95°C |
| - température en tête (T_{T}) | 13,5°C |
| - chaleur fournie au bouilleur (Q_{B}) | 16 kW |
| - pression | 12 bars absolus. |

Ces conditions de fonctionnement ont permis d'obtenir en tête un flux de F32 et d'HCI et en pied un flux de F30, F31 et HF tels que :
- les teneurs molaires en F32 et HCI du flux de F30, F31 et HF recyclés étaient inférieures à 1 % pour F32 et 100 ppm pour HCI,
- les teneurs molaires en F30, F31 et HF dans le flux de F32 et HCI étaient inférieures à 2000 ppm pour le F30, 2 % pour le F31 et 2 % pour l'HF.

La productivité de l'exemple 3, de l'ordre de 700 g/h/l de F32, n'a pas pu être atteinte. Après 60 heures de fonctionnement, les performances observées sont les suivantes :
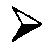 Conversion du F30 par passe : 45 %.
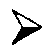 Sélectivité en F32 par passe : 96 %.

Dans le flux des produits de réaction récupéré en tête de la colonne de distillation, les impuretés principales étaient les suivantes : F23 (1,8 % mol), F22 (0,2 % mol).
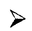 Productivité en F32 maximale atteinte = 415 g/h/litre de catalyseur.
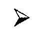 Teneur en eau dans le flux recyclé : plusieurs analyses du flux recyclé ont été effectuées tout au long de cet essai afin de déterminer sa teneur en eau (méthode karl Fischer). Les résultats ont montré qu'il n'y avait pas d'accumulation d'eau et que la teneur en H₂O dans le flux recyclé était inférieure à 300 ppm en poids.
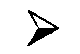 Teneur en impuretés organiques dans le recyclat. Les analyses ont montré qu'il y avait accumulation d'impuretés dans la boucle. Cette teneur est supérieure à 3 % pds au bout de 100 heures de marche. Les impuretés principales sont la série F20, le F130 et le F131.

## Revendications

1. Procédé continu de fabrication du difluorométhane (F32) à partir de chlorure de méthylène (F30) et d'acide fluorhydrique en phase gazeuse en présence d'un catalyseur de fluoration, caractérisé en ce que l'on effectue la réaction en présence de chlore, que l'on soumet le flux gazeux sortant du réacteur à une distillation pour séparer en tête un flux contenant la quasi-totalité de l'acide chlorhydrique et au moins 90 % du F32 produits par la réaction et en pied un flux contenant au moins 90 % des réactifs non transformés (F30, F31 et HF) présents dans le flux gazeux sortant du réacteur, et on recycle directement au réacteur le flux récupéré en pied de distillation, sans aucune opération de purification.

2. Procédé selon la revendication 1, dans lequel la réaction et la distillation sont menées sous une pression allant de 1 à 30 bars absolus, de préférence sous une pression comprise entre environ 10 et 15 bars absolus.

3. Procédé selon la revendication 1 ou 2, dans lequel on utilise un catalyseur à base de chrome, massique ou supporté.

4. Procédé selon l'une des revendications 1 à 3, dans lequel la réaction de fluoration est effectuée à une température comprise entre 220 et 400°C, de préférence entre 240 et 350°C.

5. Procédé selon l'une des revendications 1 à 4, dans lequel le temps de contact est compris entre 0,1 et 60 secondes, de préférence entre 1 et 20 secondes.

6. Procédé selon l'une des revendications 1 à 5, dans lequel le rapport molaire HF/Organiques en entrée de réaction est compris entre 1 et 10, de préférence entre 2 et 5.

7. Procédé selon l'une des revendications 1 à 6, dans lequel on utilise de 0,1 à 5 moles de chlore pour 100 moles d'organiques en entrée de réaction.

## Patentansprüche

1. Kontinuierliches Verfahren zur Herstellung von Difluormethan (F32) aus Methylenchlorid (F30) und Flußsäure in der Gasphase in Gegenwart eines Fluorierungskatalysators, dadurch gekennzeichnet, daß man die Reaktion in Gegenwart von Chlor durchführt, daß man den aus dem Reaktor austretenden Gasstrom einer Destillation unterzieht, um im oberen Teil einen Strom mit fast der gesamten Salzsäure und mindestens 90 % des durch die Reaktion hergestellten F32 und im unteren Teil einen Strom mit mindestens 90 % der nicht umgesetzten in dem aus dem Reaktor austretenden gasförmigen Strom enthaltenen Reaktanden (F30, F31 und HF) abzutrennen, und daß man den im unteren Destillationsteil abgetrennten Strom ohne irgendeine Reinigung dem Reaktor direkt wieder zuführt.

2. Verfahren nach Anspruch 1, in dem die Reaktion und die Destillation unter einem Absolutdruck von 1 bis 30 bar, vorzugsweise unter einem Absolutdruck zwischen ungefähr 10 und 15 bar, durchgeführt werden.

3. Verfahren nach Anspruch 1 oder 2, in dem man einen Massen- oder Trägerkatalysator auf Chrombasis verwendet.

4. Verfahren nach einem der Ansprüche 1 bis 3, in dem die Fluorierungsreaktion bei einer Temperatur zwischen 220 und 400 °C, vorzugsweise zwischen 240 und 350 °C, durchgeführt wird.

5. Verfahren nach einem der Ansprüche 1 bis 4, in dem die Verweilzeit zwischen 0,1 und 60 Sekunden, vorzugsweise zwischen 1 und 20 Sekunden, liegt.

6. Verfahren nach einem der Ansprüche 1 bis 5, in dem das Molverhältnis HF/ organische Verbindungen zu Beginn der Reaktion zwischen 1 und 10, vorzugsweise zwischen 2 und 5, liegt.

7. Verfahren nach einem der Ansprüche 1 bis 6, in dem man zu Beginn der Reaktion 0,1 bis 5 mol Chlor auf 100 mol organische Verbindungen verwendet.

## Claims

1. Continuous process for the manufacture of difluoromethane (F32) from methylene chloride (F30) and hydrofluoric acid in the gas phase in the presence of a fluorination catalyst, characterized in that the reaction is carried out in the presence of chlorine, in that the gas flow exiting from the reactor is subjected to a distillation in order to separate, at the top, a flow containing virtually all the hydrochloric acid and at least 90% of the F32 produced by the reaction and, at the bottom, a flow containing at least 90% of the unconverted reactants (F30, F31 and HF) present in the gas flow exiting from the reactor and in that the flow recovered at the distillation bottom is recycled directly to the reactor, without any purification operation.

2. Process according to Claim 1, in which the reaction and the distillation are carried out under a pressure ranging from 1 to 30 bar absolute, preferably under a pressure of between approximately 10 and 15 bar absolute.

3. Process according to Claim 1 or 2, in which a bulk or supported catalyst based on chromium is used.

4. Process according to one of Claims 1 to 3, in which the fluorination reaction is carried out at a temperature of between 220 and 400°C, preferably between 240 and 350°C.

5. Process according to one of Claims 1 to 4, in which the contact time is between 0.1 and 60 seconds, preferably between 1 and 20 seconds.

6. Process according to one of Claims 1 to 5, in which the HF/Organics molar ratio at the reaction inlet is between 1 and 10, preferably between 2 and 5.

7. Process according to one of Claims 1 to 6, in which use is made of 0.1 to 5 mol of chlorine per 100 mol of organics at the reaction inlet.
